(19) Europäisches Patentamt
European Patent Office
Office européen des brevets

(11) Veröffentlichungsnummer: **0 019 586**
**A1**

(12) # EUROPÄISCHE PATENTANMELDUNG

(21) Anmeldenummer: 80810151.3

(22) Anmeldetag: 06.05.80

(51) Int. Cl.³: A 61 K 45/06
A 61 K 31/505
//(A61K31/505, 31/415),
(A61K31/505, 31/19), (A61K31/505,
31/22)

(30) Priorität: 11.05.79 CH 4415/79

(43) Veröffentlichungstag der Anmeldung:
26.11.80 Patentblatt 80/24

(84) Benannte Vertragsstaaten:
BE CH DE FR GB IT LI LU NL SE

(71) Anmelder: CIBA-GEIGY AG
Patentabteilung Postfach
CH-4002 Basel(CH)

(72) Erfinder: White, Alan M.,Dr.
14, Newlands Road
Horsham, West Sussex(GB)

(72) Erfinder: Sallmann, Alfred, Dr.
Joachimsackerstrasse 12
CH-4103 Bottmingen(CH)

(54) **Neue antithrombotische Kombinationspräparate.**

(57) Pharmazeutische Kombinationspräparate, enthaltend als Wirkstoffkomponenten eine Verbindung der Formel

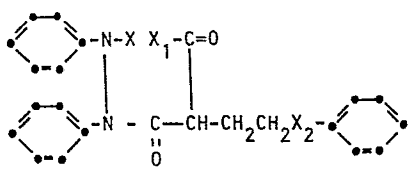

(1)

worin X Wasserstoff, $X_1$ gegebenenfalls veräthertes Hydroxy und $X_2$ Thio oder Sulfinyl bedeutet, oder worin X und $X_1$ gemeinsam eine Bindung darstellen und $X_2$ Thio ist, oder ein pharmazeutisch verwendbares Salz derselben mit einer Base und das 2,6-Bis(diäthanolamino-4, 8-dipiperidino-pyrimido-[5, 4-d]pyrimidin oder 2,6-Bis-(diäthanolamino)- 4-piperidino-pyrimido-[5, 4-d]-pyrimidin oder ein pharmazeutisch verwendbares Säureadditionssalz davon, haben einer Addition der Wirkungen der Einzelkomponenten überlegene antithrombotische Eigenschaften.

EP 0 019 586 A1

4-12350 /+

Neue antithrombotische Kombinationspräparate

Die vorliegende Erfindung betrifft neue antithrombotisch wirkende pharmazeutische Kombinationspräparate, die als pharmakologische Wirkstoffe eine die Thrombocytenfunktion regulierende Verbindung (A) und eine die Herzkranzgefässe erweiternde Verbindung (B) enthalten.

Die neuen pharmazeutischen Präparate eignen sich in erster Linie zur prophylaktischen und therapeutischen Behandlung thrombotischer Erkrankungen verschiedenster Art und unterschiedlicher Genese, insbesondere solcher der Coronararterien. Sie können prophylaktisch insbesondere zur Regulierung gestörter Thrombocytenfunktionen, vor allem zur Verhinderung des plötzlichen Herztodes "sudden death" von Coronarinfarktpatienten, und therapeutisch bei verschiedenen Erkrankungen, bei denen eine Störung der Thrombocytenfunktion den auslösenden oder einen begleitenden Faktor darstellt, z.B. bei Coronarinsuffizienz und Coronarinfarkt, eingesetzt werden.

Verbindungen mit die Thrombocytenfunktionen regulierenden Eigenschaften (A) bewirken insbesondere eine Reduktion der Umsatz-, d.h. der Produktions- und Absterberate, der Thrombocyten und verlängern

deren Ueberlebenszeit, insbesondere bei einer krankheitsbedingten Verkürzung derselben, beispielsweise aufgrund thrombotischer Störungen oder bösartiger Tumore. Zudem vermindern sie eine pathologisch bedingte erhöhte Adhäsivität und Aggregationstendenz der Blutplättchen.

Verbindungen mit die Herzkranzgefässe erweiternden Eigenschaften (B) bewirken insbesondere eine Erweiterung verengter Coronarkapillargefässe, wie sie z.B. bei primären und gewissen sekundären Hyperlipidämien auftreten. Damit gestatten sie, durch Erhöhung des Coronargefässlumens die Blutversorgung des Herzmuskels zu verbessern und so eine der klinisch bedeutsamsten Ursachen für die obengenannten Erkrankungen, insbesondere des Coronarinfarktes, prophylaktisch und therapeutisch günstig zu beeinflussen.

Durch die kombinierte Anwendung der beiden Wirkstofftypen (A+B) wird ein ausgeprägter synergistischer Effekt erzielt, da sich die Wirkungen der pharmakologisch wirksamen Komponenten (A) und (B) in überraschender Weise derart ergänzen, dass die Wirkung der Kombination (A+B) höher ist als einer Addition der Einzelwirkungen der Komponenten (A) und (B) entspricht. Als besonderer Fortschritt ist dabei anzusehen, dass die beiden Komponenten A und B niedriger dosiert werden können als bei einer Monotherapie und damit deren bekannten oder möglichen Nebenwirkungen unterdrückt werden können, und dass die neuen Kombinationen (A+B) gleichzeitig auf zwei verschiedenen Wegen, nämlich durch die Regulierung sowohl der gestörten Thrombocytenfunktionen als auch der gestörten Coronargefässfunktion, die klinisch bedeutsamsten Risikofaktoren der genannten Pathologien, namentlich des Coronarinfarktes, günstig beeinflussen.

Die therapeutische Wirksamkeit der erfindungsgemässen Kombinationspräparate kann in Tierversuchen anhand der Verlängerung Schwanzblutungszeit gezeigt werden, die die Hemmwirkung der Testsubstanz auf die Störung der Blutblättchenfunktion und ihre gefässerweiterde Wirkung wiedergibt, ebenso an Tiermodellen, in denen in vivo eine Cyclooxygenase-abhängige Blutblättchenaggregation erfolgt.

Für die Messung der Blutungszeit ist bei der Ratte die Methode von Dejana et al., Thrombosis Research 15, 199-207 (1979) besonders geeignet. Dabei wird männlichen Ratten von 180-200 g Körpergewicht der Schwanz etwa 2 mm vor der Schwanzspitze durchtrennt und der Schwanz unmittelbar danach in isotonische Kochsalzlösung von 37°C eingetaucht. Die Blutungszeit wird gemessen vom Augenblick der Durchtrennung bis zum vollständigen Stillstand der Blutung. Die Testsubstanzen werden dabei 1 Stunde vor der Messung als 5% Suspension in Carboxymethylcellulose intraperitoneal verabfolgt. Normalerweise werden jeweils 10 Versuchstiere verwendet.

Die für die Messung der Cyclooxygenase-abhängigen Blutplättchenaggregation bestgeeignete Versuchsmethode bedient sich der Arthus-Reaktion in der von Butler et al. (Thrombosis Research 15, 319-340 [1979] beschriebenen Form. Dazu werden gegen Ovalbumin immunisierte Meerschweinchen (300-400 g Körpergewicht, Dunkin-Hartley-Zuchtstamm) verwendet. Zur Immunisierung werden anfangs 0,5 mg Ovalbumin als 0,1%-ige Lösung in Phosphatpuffer vom pH 7,4 an 4 Stellen des Rückens subcutan verabfolgt. 2 Wochen später werden die Tiere mit Mepyramine (1 mg/kg i.p. in 0,5 ml pysiologischer Kochsalzlösung) und einer weiteren Einzeldosis der Ovalbumin-Suspension behandelt. Nach einer weiteren Woche werden die nunmehr immunisierten Versuchstiere in 3 Gruppen aufgeteilt. Eine Gruppe dient als Kontrollgruppe. Die zweite wird dem Antigen-Challenge ausgesetzt und 20 Minuten später deren Blutplättchenkennzahl bestimmt. Die dritte Gruppe wird zusätzlich 1 Stunde vor dem Challenge intravenös mit der Testsubstanz behandelt. In einer weiteren Cyclooxygenase-abhängigen Versuchanordnung kann die Normalisierung der Thrombocytenfunktion am Affen gezeigt werden. Dazu wird männlichen Pavianen mit einem Gewicht

von 8-12 kg unter Narkose ein A-V-Shunt zwischen Arteria und Vena femoralis angelegt. Dieser Shunt ist ca. 50 cm lang und besteht aus einem besonders präparierten Silastikschlauch. Der Fremdkörperreiz dieser körperfremden Oberfläche bewirkt einen erhöhten Thrombocytenverbrauch, denn der Organismus ist bestrebt, diesen "Defekt" abzudecken. Der Thrombocytenverbrauch kann ermittelt werden, indem man dem Versuchtier mit Chrom 5α radioaktiv markierte Thrombocyten injiziert und deren Verschwinden aus der Blutbahn 2 x täglich messend verfolgt. Bei einem normalen Thrombocytenumsatz beträgt die Lebensdauer der Thrombocyten etwa 5 Tage, bei Tieren mit einem eingesetzten Shunt verkürzt sich die mittlere Thrombocytenlebenszeit als Ausdruck des erhöhten Thrombocytenumsatzes auf etwa 2-2,5 Tage. Bei der Substanzprüfung wird die zu prüfende Substanz über den Versuchszeitraum von 4-5 Tagen den wachen Tieren 4 mal täglich oral verabreicht.

Als weitere Cyclooxygenase-abhängige Versuchsanordnung kommt in vitro die Hemmung der Prostaglandinsynthese aus Arachidonsäure nach Prostaglandins 7, 123 (1974) in Betracht.

Mit den neuen pharmazeutischen Präparaten werden für praktische Zwecke zur prophylaktischen und therapeutischen Behandlung thrombotischer Erkrankung der Coronargefässe vorzüglich geeignete Arzneimittel zur Verfügung gestellt, die gute Langzeitverträglichkeit aufweisen, keine störenden Nebenwirkungen zeigen und durch die auf ihren spezifischen Wirkungsmechanismus beruhenden starken Verminderung des Coronarinfarktrisikos einen bedeutenden Fortschritt in der Behandlung thrombotischer Coronarerkrankungen und ebenfalls in der Unterdrückung der Metastasierung bösartiger Tumore darstellen.

Die Thrombocytenfunktionen regulierenden Verbindungen (A) sind 2-Phenyläthylthio-N,N'-diphenyl-malonsäurehydrazide der Formel

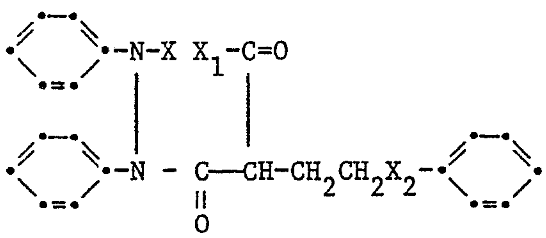

(I)

worin X Wasserstoff, $X_1$ gegebenenfalls veräthertes Hydroxy und $X_2$ Thio oder Sulfinyl bedeutet, oder worin X und $X_1$ gemeinsam eine Bindung darstellen und $X_2$ Thio ist, und pharmazeutisch verwendbare Salze derselben mit Basen.

Die Verbindungen der Formel I, worin X und $X_1$ gemeinsam eine Bindung darstellen und $X_2$ Thio ist, d.h. 1,2-Diphenyl-4-(2-phenylthioäthyl)-pyrazolidin-3,5-dion, ist bekannt und nebst einem Verfahren zu seiner Herstellung in Helv. Chem. Acta 44, 232-237 (1961) beschrieben. Ferner ist, z.B. aus der Schweiz. Med. Wochenschrift 84, 1315-1318 (1954), bekannt, dass die genannte Verbindung und ihre pharmazeutisch verwendbaren Salze analgetisch und antiphlogistisch wirksam sind und dementsprechend zur Behandlung rheumatischer Erkrankungen, wie der rheumatischen Arthritis, verwendet werden können.

Die Verbindungen der Formel I, worin X Wasserstoff und $X_1$ gegebenenfalls veräthertes Hydroxy ist, d.h. 2-(2-Phenylthioäthyl)- bzw. 2-(2-Phenylsulfinyläthyl)-malonsäure-N,N-diphenylmonohydrazid und ihre Ester, sind demgegenüber neu. Die genannten Monohydrazide können z.B. hergestellt werden, indem man 1,2-Diphenyl-4-(2-phenylthioäthyl)- bzw. 1,2-Diphenyl-4-(2-phenylsulfinyläthyl)-pyrazolidin-3,5-dion in üblicher Weise, beispielsweise durch Erhitzen mit verdünnter Natronlauge, unter Spaltung der in Formel I durch X und $X_1$ gemeinsam dargestellten Bindung hydrolysiert. Das so erhältliche 2-(2-Phenylthioäthyl)- bzw. (2-Phenylsulfinyläthyl)-malonsäure-N,N'-diphenyl-monohydrazid kann gewünschtenfalls in üblicher Weise, z.B. durch Behandlung mit einer Base, in ein pharmazeutisch verwendbares Salz , überführt werden. Ester derselben, d.h.

Verbindungen der Formel I, worin X Wasserstoff und $X_1$ veräthertes Hydroxy darstellt, können z.B. erhalten werden, indem man einen entsprechenden 2-(2-Phenylthioäthyl)- bzw. (2-Phenylsulfinyläthyl)-malonsäuremonoester in Gegenwart von Dicyclohexylcarbodiimid mit N,N'-Diphenylhydrazin kondensiert.

Veräthertes Hydroxy $X_1$ ist beispielsweise Niederalkoxy mit bis zu 7, vor allem mit bis zu 4, Kohlenstoffatomen, wie Methoxy, Aethoxy, Propyloxy, Isopropyloxy, Butyloxy, Sekundärbutyloxy, Isobutyloxy oder Tertiärbutyloxy, ferner Pentyloxy, Hexyloxy oder Heptyloxy.

Pharmazeutisch verwendbare Salze von Verbindungen der Formel I mit Basen sind in erster Linie pharmazeutisch verwendbare Metall- und Ammoniumsalze, wie Alkalimetall- oder Erdalkalimetallsalze, z.B. Natrium-, Kalium- oder Calciumsalze, ferner Ammoniumsalze mit Ammoniak oder geeigneten Amine. Geeignete Amine sind in erster Linie aliphatischen, cycloaliphatische, cycloaliphatisch-aliphatische oder araliphatische primäre, sekundäre oder tertiäre Mono, Di- oder Polyamide, sowie heterocyclische Basen, wie Niederalkylaminen, z.B. Triäthylamin, Hydroxyniederalkylamine, z.B. 2-Hydroxyäthylamin, 2-Hydroxy-1,1-bis(hydroxymethyl)-äthylamin oder Tris-(2-hydroxyäthyl)-amin, Niederalkylenamine, z.B. 1-Aethyl-piperidin, Cycloalkylamine, z.B. Dicyclohexylamin, oder Benzylamino, z.B. N,N'-Dibenzyläthylendiamin, ferner Basen von Pyridintyp, z.B. Pyridin, Collidin oder Chinolin.

Als die Herzkranzgefässe erweiternden Verbindungen (B) kommen das 2,6-Bis(diäthanolamino)-4,8-dipiperidino-pyrimido-[5,4-d]-pyrimidin sowie das 2,6-Bis-(diäthanolamino)-4-piperidino-pyrimido-[5,4-d]-pyrimidin und dessen pharmazeutisch verwendbare Säureadditionssalze in Betracht.

2,6-Bis(diäthanolamino)-4,8-dipiperidino-pyrimido-[5,4-d]-pyrimdin ist ebenfalls bekannt und beispielsweise im britischen Patent Nr. 807,826 beschrieben. Es wird unter dem "generic name" Dipyridamol seit langem als koronarerweiterndes Arzneimittel verwendet.

2,6-Bis(diäthanolamino)-4-piperidino-pyrimido-[5.4-d]-pyrimidin ist unter dem "generic name" Mopidamol bekannt. Pharmazeutisch verwendbare Säureadditionssalze des 2,6-Bis(diäthanolamino)-4,8-dipiperidino-pyrimido-[5,4-d]pyrimidins sind vor allem Säureadditionssalze mit pharmazeutisch verwendbaren Mineralsäuren, z.B. dessen Hydrochlorid oder Hydrobromid oder N-Cyclohexylsulfaminat, ferner Salze mit organischen Sulfonsäuren, z.B. dessen Methan- oder p-Toluolsulfonat, oder organischen Carbonsäuren, wie dessen Dicarbonsäuresalze, z.B. dessen Maleat, Maleinat oder Fumarat.

Die Erfindung betrifft in erster Linie pharmazeutische Präparate, die als Komponente A 1,2-Diphenyl-4-(2-phenylthioäthyl)-pyrazolidin-3,5-dion oder 2-(2-Phenylthioäthyl)-malonsäure-N,N'-diphenylhydrazid oder einen $C_1$-$C_4$-Alkylester oder Alkalimetallsalz davon und als Komponente B 2,6-Bis(diäthanolamino)-4,8-dipiperidino-pyrimido-[5,4-d]pyrimidin oder 2,6-Bis(diäthanolamino)-4-piperidino-pyrimido-[5,4-d]pyrimidin oder ein pharmazeutisch verwendbares Säureadditionssalz davon erhalten.

Die Erfindung betrifft namentlich pharmazeutische Präparate, die als Komponente A 1,2-Diphenyl-4-(2-phenylthioäthyl)-pyrazolidin-3,5-dion und als Komponente B 2,6-Bis(diäthanolamino)-4,8-dipiperidino-pyrimido-[5,4-d]pyrimidin enthalten.

Ein weiterer Gegenstand der vorliegenden Erfindung ist die Verwendung von pharmazeutischen Präparaten zur Behandlung thrombotischer Erkrankungen, vorzugsweise der Coronargefässe, die als pharmazeutische Wirkstoffe eine die Thrombocytenfunktionen regulierende Verbindung (A) der Formel I, vorzugsweise die vorstehend als bevorzugt bezeichneten, oder ein pharmazeutisch verwendbares Salz davon mit einer Base und als die Herzkrankzgefässe erweiternde Verbindung (B), die vorstehend genannte, oder ein pharmazeutisch verwendbares Säureadditionssalz enthalten.

Zur Herstellung der neuen pharmazeutischen Präparaten wird die Thrombocytenfunktionen regulierende Verbindung (A) mit der die Herzkranzgefässe erweiternde Verbindung (B) im Gewichtsverhältnis A:B von etwa 5:1 bis etwa 1:5, vor allem von etwa 2:1 bis etwa 1:2 miteinander kombiniert und mit bei der Herstellung von Arzneimitteln üblichen Träger- und/oder Hilfsstoffen sowie gegebenenfalls anderen Wirkstoffen zu pharmazeutischen Präparaten verarbeitet. In den als bevorzugten Erfindungsgegenstand zu betrachtenden Kombinationen von 1,2-Diphenyl-4-(2-phenylthioäthyl)-pyrazolidin-3,5-dion mit 2,6-Bis-(diäthanolamino)-4,8-dipiperidino-pyrimido-[5.4-d]-pyrimidin oder 2,6-Bis(diäthanolamino)-4-piperidino-pyrimido-[5,4-d]pyrimidin liegt das optimale Verhältnis vorzugsweise innerhalb des durch die Annäherungswerte 1,5:1 bis 1:5 abgegrenzten Bereich, wobei der Bereich von etwa 1:1 bis 1:2 als besonders günstig betrachtet wird.

Die absolute Dosierung der beiden Wirkstoffkomponenten A und B hängt ausser von der wirksamen Dosis der jeweils zu verwendenden Komponente A vor allem von der individuellen Ansprechbarkeit auf die jeweiligen Komponenten A und B ab. Allgemein enthalten die erfindungsgemässen pharmazeutischen Präparate pro Dosiseinheit jeweils etwa 10 bis etwa 100 mg der Komponente A und etwa 25 bis 150 mg der Komponente B. Die als bevorzugten Erfindungsgegenstand zu betrachtenden Kombinationspräparate mit 1,2-Diphenyl-4-(2-phenylthioäthyl)-pyrazolidin-3,5-dion und 2,6-Bis(diäthanolamino)-4,8-dipiperidino-pyrimido-[5,4-d]pyrimidin oder 2,6-Bis(diäthanolamino)-4-piperidino-pyrimido-[54-d]pyrimidin enthalten vorzugsweise von etwa 10 bis etwa 75 mg, insbesondere von etwa 20 bis etwa 60 mg der erstgenannten und von etwa 25 bis 75 mg der letztgenannten Verbindung.

Als Ausgangspunkt für die Posologie ist zwei- bis viermal-, vorzugsweise dreimal-täglich Verabfolgung einer Dosiseinheit angezeigt.

Die neuen pharmazeutischen Präparate sind insbesondere zur enteralen, vor allem oralen Applikation bestimmt. Vorzugsweise handelt es sich um Tabletten, Dragées oder Kapseln, in zweiter Linie um Suppositorien, die jeweils neben den erfindungsgemäss zu verwendenden Wirkstoffen für die jeweilige Applikationsform übliche Hilf- und Trägerstoffe enthalten, die die Verarbeitung der Wirkstoffe in die erfindungsgemässen Präparationen erleichtern und eine gesteuerte Freisetzung der Wirkstoffe ermöglichen. Die neuen pharmazeutischen Präparate können dabei auch als Punkttabletten oder Punktdragées formuliert werden. Diese bestehen im Prinzip aus einem die eine Wirkstoffkomponente enthaltende Tabletten- bzw. Drageéskern, einem die andere Wirkstoffkomponente enthaltenden sogenannten Punkt, der auf den Kern aufgepresst wird, und erforderlichenfalls üblichen Trenn-, Deck- und/oder Schutzschichten.

Der Kern ist vorzugsweise aus dem Wirkstoff bzw. einen diesen enthaltenden Wirkstoff-Hilfsstoffgemisch und gegebenenfalls Hilfs-, Träger- und/oder Hüllstoffen zusammengesetzt. Ein Wirkstoff-Hilfsstoff-Gemisch ist z.B. ein entsprechendes Granulat, das sich zur Verarbeitung zu Tabletten, Dragées oder Kapseln eignet. Ein solches Granulat enthält ausser der Wirkstoff u.a. Verdünnungs- oder Füllstoffe, wie Zucker, z.B. Lactose oder Saccharose, oder Zuckeralkohole, z.B. Mannit oder Sorbit, Cellulosepräparate und/oder Calciumhydrogenphosphat, ferner Fliessreguliermittel, wie Talk oder kollodale Kieselsäure, Gleit-, Schmier- bzw. Antiklebemittel, wie Stearinsäure oder Salze davon, z.B. Magnesium- oder Calciumstearat, und/oder Polyäthylenglykol, ferner Glyceride, wie hydriertes Baumwollsamen- oder hydriertes Rizinusöl. Ein solches Granulat kann in an sich bekannter Weise, gegebenenfalls unter Verwendung von Befeuchtungsmitteln, wie Wasser oder organischen Lösungsmitteln, z.B. Aethanol, oder Gemischen davon hergestellt werden.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man Tabletten bzw. Dragées-Kerne zur oralen Anwendung erhalten, indem man die Wirkstoffe mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Als Trägerstoffe kommen beispielsweise in Betracht: Zucker, z.B. Lactose, Saccharose, Mannit oder Sorbit, übliche Cellulosepräparationen, Calciumphosphate, z.B. Tricalcium- oder Calciumhydrogenphosphat, Bindemittel, wie Stärkekleister, z.B. auf Basis von Mais-, Weizen-, Reis- oder Kartoffelstärke, Gelatine, Tragakanth, Methylcellulose, Natriumcarboxylcellulose und/oder Polyvinylpyrroliden, ferner Carboxymethylstärke, quervernetztes Polyvinylpyrrolidon, Agar, Alginsäure bzw. ein Alginat, z.B. Natriumalginat, und/oder, wenn erwünscht, Sprengmittel, wie die obengenannten Stärken. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konznetrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Durch Einarbeiten eines oder mehrerer Wirkstoffe in ein geeignetes Trägermaterial, das eine langsame Abgabe des oder der Wirkstoffe bewirkt, kann die Wirkungsdauer einer oder mehrerer an sich kurz wirksamen Komponenten verlängert werden. Den Tabletten oder Dragée-Ueberzügen können Farbstoffe oder Pigmente, z.B. zur Identifizierung oder zur Kennzeichnung verschiedener Kombinationen von Wirkstoffdosen, beigefügt werden.

Weitere oral anwendbare pharmazeutische Präparate sind Steckkapseln aus Gelatine, sowie weiche, geschlossende Kapseln aus Gelatine und einem Weichmacher, wie Glycerin oder Sorbitol. Die Steckkapseln können die Wirkstoffe in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln sind die Wirkstoffe vorzugsweise in geeigneten Flüssigkeiten, wie fette Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination der Wirkstoffe mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die aus einer Kombination der Wirkstoffe mit einer Grundmasse bestehen; als Grundmasse kommen z.B. Polyäthylenglykole oder Paraffinkohlenwasserstoffe in Frage.

Hilfsmittel sind in erster Linie Fliessregulier- und/oder Schmiermittel, z.B. Kieselsäure, Talk, Stearinsäure oder ein Salz davon, z.B. Magnesium- oder Calciumstearat, und/oder Polyäthylenglycol.

Gewünschtenfalls kann man erhaltene Tabletten bzw. Punkttabletten zur Herstellung von sogenannten Lacktabletten oder Dragées mit geeigneten, gegebenenfalls Magensaft-resistenten Tabletten- bzw. Dragées-Ueberzügen versehen, wobei man u.a. konzentrierte Zurckerlösungen, welche gegebenenfalls arabischen Gumme, Talk, Polyvinylpyrrolidon, Polyäthylenglycol und/oder Titandioxid enthalten, Lacklösungen in geeigneten organischen Lösungsmittel doer Lösungsmittelgemischen oder, zur Herstellung von Magensaft-resistenten Ueberzügen,

Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Kombination von Wirkstoffdosen, beigefügt werden.

Wie oben ausgeführt, werden die erfindungsgemässe enteral, vorzugsweise oral, verabreichbaren pharmazeutischen Präparate in an sich bekannter Weise hergestellt; die Herstellung wird in den nachfolgenden Beispielen näher erläutert.

Beispiel 1: Lacktabletten enthaltend 25 mg 1,2-Diphenyl-4-(2-phenylthioäthyl)-pyrazolidin-3,5-dion und 75 mg 2,6-Bis (diäthanolamino)-4,8-dipiperidino-pyrimido-[5,4-d]pyrimidin können wie folgt hergestellt werden:

Zusammensetzung (für 10'000 Tabletten),

| | |
|---|---|
| 1,2-Diphenyl-4-(2-phenylthioäthyl)-pyrazolidin-3.5-dion | 250 g |
| 2,6-Bis(diäthanolamino)-4,8-dipiperidino-pyrimido-[5,4-d]pyrimidin | 750 g |
| Maisstärke | 880 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Die Lacktabletten werden wie folgt hergestellt (für 10'000 Tabletten):

Das Gemisch von 250 g 1,2-Diphenyl-4-(2-phenylthioäthyl)-pyrazolidin-3,4-dion, 750 g 2,6-Bis(diäthanolamino)-4,8-dipiperidino-pyrimido-[5,4-d]pyrimidin, Maisstärke und 200 g kolloidaler Kieselsäure wird mit einem Stärkekleister aus 450 g Maisstärke und 2,2 kg entmineralisiertem Wasser zu einer feuchten Masser verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Machenweite gedrückt, mit einer zum Voraus gesiebten Mischung (1 mm Sieb) von 130 g Maisstärke, 20 g Magnesiumstearat, 50 g Stearinsäure und 250 g Natriumcarboxymethylstärke gemischt und zu schwach gewülbten Tabletten von 6 mm Durchmesser gepresst.

Die nach dem obigen Verfahren herstellbaren Tabletten werden wie folgt mit einer Lackschicht versehen:

Die Presslinge werden in einem Dragierkessel von 45 cm Druchmesser mit einer Lösung von 20 g Schellack und 40 g Hydroxypropylmethylcelluslose (niedere Viskosität) in 110 g Methanol und 1350 g Methylenchlorid durch gleichmässiges Aufsrpühen während 30 Minuten überzogen; dabei wird druch gleichzeitiges Einblasen von Luft von 60° getrocknet.

Anstelle des 1,2-Diphenyl-4-(2-phenylthioäthyl)-pyrazolidin-3,5-dions kann auch die entsprechende Menge eines seiner Salze, z.B. des Natriumsalzes, verwendet werden.

Beispiel 2: Lacktabletten, enthaltend 50 mg 1,2-Diphenyl-4-(2-phenylthioäthyl)-pyrazolidin-3,5-dion und 50 mg 2,6-Bis(diäthanolamino)-4,8-dipiperidino-pyrimido-[5,4-d]pyrimidin oder die entsprechende Menge des Natriumsalzes der erstgenannten Verbindung, können wie folgt hergestellt werden:

Zusammensetzung (für 20'0C Tabletten):

| | |
|---|---|
| 1,2-Diphenyl-4-(2-phenylthioäthyl)-pyrazolidin-3.5-dion | 1000 g |
| 2,6-Bis(diäthanolamino)-4,8-dipiperidino-pyrimido-[5.4-d]pyrimidin | 1000 g |
| Maisstärke | 2400 g |
| Kolloidale Kieselsäure | 600 g |
| Magnesiumstearat | 60 g |
| Stearinsäure | 140 g |
| Natriumcarboxymethylstärke | 700 g |
| Wasser | q.s. |

Die Lacktabletten werden wie folgt hergestellt (für 20'000 Tabletten):

Die Mischung von 1,0 kg 1,2-Diphenyl-4-(2-phenylthioäthyl)-pyrazolidin-3,5-dion und 1.0 kg 2.6-Bis(diäthanolamino)-4,8-dipiperidino-pyrimido-[5,4-d]pyrimidin, 750 g Maisstärke und 600 g kolloidale Kieselsäure wird mit einem Stärkekleister aus 1250 g Maisstärke und 6,4 kg entmineralisiertem Wasser zu einer feuchten Masse verarbeitet. Diese wird durch ein Sie von 3 mm Maschenweite getrieben und während 30 Minuten bei 45° im Wirbelschichttrockner getrocknet. Das trockene Granulat wird durch ein Sieb von 1 mm Maschenweite getrieben, mit einer zum Voraus gesiebten Mischung (1 mm Sieb) von 400 g Maisstärke, 60 g Magnesiumstearat, 140 g Stearinsäure und 700 g Natriumcarboxymethystärke gemischt und zu schwach gewölbten Tabletten von 6 mm Durchmesser gepresst.

Die Lackschitcht wird wie folgt auf die so erhältlichen Tabletten aufgetragen.

5000 der obigen Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch kontinuierliches Aufsprühen während 30 Minuten überzogen; dabei wird durch gleichzeitiges Einblasen von Warmluft von 60° getrocknet.

Anstelle des 1,2-Diphenyl-4-(2-phenylthioäthyl)-pyrazolidin-3,5-dion kann man auch die entsprechende Menge eines seiner Salze, z.B. des Natriumsalzes, verwenden.

Beispiel 3: Tabletten, enthaltend 50 mg 1,2-Diphenyl-4-(2-phenylthioäthyl)-pyrazolidin-3,5-dion und 75 mg 2,6-Bis(diäthanolamino)-4,8-dipiperidino-pyrimido-[5,4-d]pyrimidin oder die entsprechende Menge des Natriumsalzes der erstgenannten Verbindung können z.B. wie folgt hergestellt werden.

Zusammensetzung (für 1 Tablette)

| | |
|---|---|
| 1,2-Diphenyl-4-(2-phenylthioäthyl)-pyrazolidin-3,5-dion | 50 mg |
| 2,6-Bis(diäthanolamino)-4,8-dipiperidino-pyrimido-[5,4-d]pyrimidin | 75 mg |
| Lactose | 75 mg |

| | |
|---|---|
| Weizenstärke | 150 mg |
| Kolloidale Kieselsäure | 24 mg |
| Magnesiumstearat | 4 mg |
| Talk | 22 mg |
| Wasser | q.s. |

Die Tabletten werden wie folgt hergestellt:

Die Wirkstoffe werden mit einem Teil der Weizenstärke, mit der Lactose und der kolloidalen Kieselsäure vermischt und das Gemisch durch ein Sieb getrieben. Ein weiterer Teil der Weizenstärke wird mit der fünffachen Menge Wasser auf dem Wasserbad verkleistert und die obige Pulvermischung mit diesem Kleister angeknetet, bis eine schwach plastische Massen entstanden ist. Die plastische Masse wird durch ein Sieb von etwa 3 mm Maschenweite gedrückt, getrocknet und das trockene Granulat nochmals durch ein Sieb getrieben. Darauf werden die restliche Weizenstärke, der Talk und das Magnesiumstearat zugemischt und die erhaltene Mischung zu schwach gewölbten Tabletten von je 0,4 g verpresst.

Beispiel 4: Punkttabletten enthaltend 20 mg 1,2-Diphenyl-4-(2-phenylthioäthyl)-pyrazolidin-3,5-dion oder die entsprechende Menge seines Natriumsalzes im Punkt und 75 mg 2,6-Bis(diäthanolamino)-4,8-dipiperidino-pyrimido-[5,4-d]pyrimdin im Kern werden wie folgt hergestellt:

Zusammensetzung (für 1 Tablette):

| | | |
|---|---|---|
| Punkt: | 1,2-Diphenyl-4-(2-phenylthioäthyl)-pyrazolidin-3,5-dion | 20 mg |
| | Weizenstärke | 5 mg |
| | Talk | 2 mg |
| | Magnesiumstearat | 1 mg |
| | Lactose | 5 mg |

| | | |
|---|---|---|
| Kern: | 2,6-Bis(diäthanolamino)-4,8-dipiperidino-pyrimido-[5,4-d]pyrimidin | 75 mg |
| | Hydroxymethylcellulose niederer Viskosität | 15 mg |
| | Hydriertes Baumwollsamenöl | 15 mg |
| | Kolloidales Siliziumhydroxid | 1 mg |
| | Magnesiumstearat | 5 mg |
| | Lactose | 200 mg |

Herstellung

Zur Herstellung von 1000 Kernen wird ein Gemisch von 75 mg 2,6-Bis(diäthanolamino)-4,8-dipiperidino-pyrimido-[5,4-d]pyrimidin, der Lactose, der kolloidalen Kieselsäure und der Hydroxymethylcallulose mit 150 g entmineralisiertem Wasser zu einer feuchten Masse verarbeitet. Diese wird durch ein Sieb von 3 mm Maschenweite granuliert, bei 45°C 30 Minuten getrocknet und dann durch ein Sieb von 0,6 bis 0,8 mm Maschenweite weiterzerkleinert. Nach Zumischen des hydrierten Baumwollsamenöls (in Pulverform) und des Magnesiumstearats werden gewölbte Kernpresslinge gepresst.

Zur Herstellung von 1000 Punkten werden 20 g 1,2-Diphenyl-4-(2-phenylthioäthyl)-pyrazolidin-3,5-dion mit der feingemahlenen Lactose und einem Teil der Weizenstärke vermischt und die Mischung durch ein engmaschiges Sieb getrieben. Der Rest der Weizenstärke wird mit der fünffachen Wassermenge auf dem Wasserbad verkleistert und die gemäss vorstehenden Angeaben erhaltene Pulvermischung mit diesem zu einer plastischen Masse angeknetet. Die so erhaltene Masse wird durch ein Sieb von 3 mm Maschenweite granuliert, getrocknet, durch ein Sieb von 0,6-0,8 mm Maschenweite getrieben, mit dem Talk und dem Magnesiumstearat innig vermischt und zu Punktpresslingen verpresst.

Zur Herstellung der Punkttabletten wird der Punktpressling sodann auf den Kern aufgepresst und zwar so, dass in der fertigen Punkttablette eine Oberfläche des aufgepressten Punktes von aussen sichtbar ist.

Beispiel 5: Dragées, enthaltend 50 mg 2,6-Bis(diäthanolamino)-4,8-dipiperidino-pyrimido-[5,4-d]pyrimidin und 75 mg 1,2-Diphenyl-4-(2-phenylthioäthyl)-pyrazolidin-3,5-dion, bzw. das Natriumsalz davon können z.B. folgendermasser hergestellt werden:

1000 g der z.B. nach Beispiel 3 erhältlichen Tabletten werden in üblicher Weise in zwei Stufen mit einem zuckerhaltigen Sirup dragiert. Als solche wird in der ersten Stufe ein Sirup erhalten aus einem Teil Zucker und zwei TEilen Wasser unter Zusatz von Talk (18%), Polyvinylpyrrolidon (1,5%) und Polyäthylenglykol 6000 (1%) und ein der zweiten Stufe ein reiner Zuckersirup verwendet.

Beispiel 6: Kapseln enthaltend 60 mg 1,2-Diphenyl-4-(2-phenylthioäthyl)-pyrazolidin-3,5-dion oder dessen Natriumsalz und 25 mg 2,6-Bis(diäthanolamino)-4,8-dipiperidino-pyrimido-[5,4-d]pyrimidin, können z.B. folgendermassen hergestellt werden:

Zusammensetzung (für 1 Tablette):

Pulvermischung:

| | |
|---|---|
| 1,2-Diphenyl-4-(2-phenylthioäthyl)-pyrazolidin-3,5-dion | 60,8 mg |
| Maisstärke | ca. 158,0 mg |
| Milchzucker plv. | ca. 100,0 mg |
| Magnesiumstearat | 2,0 mg |
| | 320,0 mg |

Kern Ø 6 mm:

| | |
|---|---|
| 2,6-Bis(diäthanolamino)-4,8-dipiperidino-pyrimido-[5,4.d]pyrimidin | 25,0 mg |
| Kollidon $25^R$ (Polyvinylpyrrolidon) | 2,5 mg |
| Formaldehydgelatine | 6,5 mg |
| Magnesiumstearat | 1,0 mg |
| Milchzucker | 25,0 mg |

Ueberzug:

| | | |
|---|---|---|
| Talkum | ca. | 13,4 mg |
| Zucker | ca. | 4,2 mg |
| Gummi arabicum | ca. | 2,4 mg |
| | ca. | 80,0 mg |

Gesamtfüllgewicht: Pulver/Kern ca. 400 mg.

Hartgelatin Kapselhüllen der Grösse 0 werden mit je einem Kern und 300 mg Pulvermischung gefüllt, verschlossen und mit je etwa 100 mg Ueberzugsmasse überzogen.

Beispiel 7: In analoger Weise wie in den Beispielen 1 bis 6 beschrieben kann man auch pharmazeutische Präparate herstellen, die statt des 1,2-Diphenyl-4-(2-phenylthioäthyl)-pyrazolidin-3,5-dions oder seines Natriumsalzes die entsprechende Menge 2-(2-Phenylthioäthyl)-malonsäure-N,N'-diphenyl-hydrazid oder deren Natriumsalz bzw. ein 2-(2-Phenylthioäthyl)-malonsäureniederalkylester-N,N'-diphenyl-hydrazid enthalten.

Beispiel 8: In analoger Weise wie in den Beispielen 1 bis 6 beschrieben kann man auch pharmazeutische Präparate herstellen, die statt des 1,2-Diphenyl-4-(2-phenylthioäthyl)-pyrazolidin-3,5-dions oder seines Natriumsalzes die entsprechende Menge 2-(2-Phenylsulfinyläthyl)-malonsäure-N,N'-diphenyl-hydrazid oder deren Natriumsalz bzw. ein 2-(2-Phenylsulfinyläthyl)-malonsäure-niederalkylester-N,N'-diphenyl-hydrazid enthalten.

Beispiel 9: In analoger Weise wie in den Beispielen 1-8 beschrieben, kann man auch pharmazeutische Präparate herstellen, die statt des 2,6-Bis(diäthanolamino)-4,8-dipiperidino-pyrimido-[5,4-d]-pyrimidins die entsprechende Menge 2,6-Bis(diäthanolamino)-4-piperidino-pyrimido-[5,4-d]pyrimidin enthalten.

Patentansprüche

1. Pharmazeutische Kombinationspräparate, die als pharmakologische Wirkstoffe eine die Thrombocytenfunktion regulierende Verbindung (A) und eine die Herzkranzgefässe erweiternde Verbindung (B) enthalten.

2. Pharmazeutische Präparate gemäss Anspruch 1, enthaltend als Wirkstoffkomponent (A) eine Verbindung der Formel

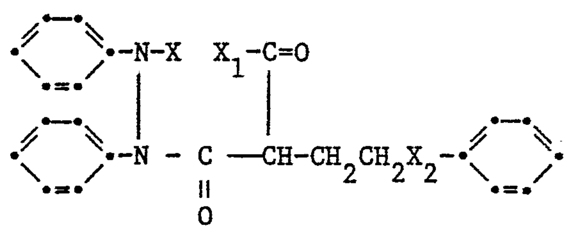

(I)

worin X Wasserstoff, $X_2$ gegebenenfalls veräthertes Hydroxy und $X_2$ Thio oder Sulfinyl bedeutet, oder worin X und $X_1$ gemeinsam eine Bindung darstellen und X Thio ist oder ein pharmazeutisch verwendbares Salz derselben mit einer Base und/oder als Wirkstoffkomponente (B) das 2,6-Bis(diäthanolamino)-4,8-dipiperidino-pyrimido-[5,4-d]pyrimidin oder das 2,6-Bis(diäthanolamino)-4-piperidino-pyrimido-[5,4-d]pyrimidin oder ein pharmazeutisch verwendbares Säureadditionssalz davon.

3. Pharmazeutische Präparate gemäss Anspruch 1, enthaltend als Wirkstoffkomponent (A) eine Verbindung der Formel

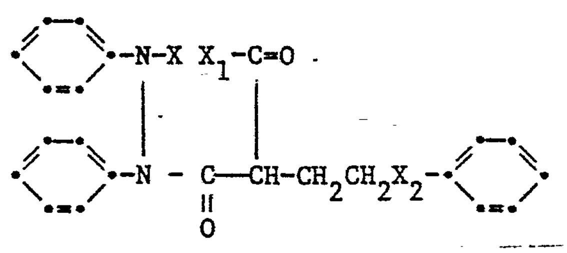

(I)

worin X Wasserstoff ist und $X_1$ gegebenenfalls veräthertes Hydroxy bedeutet, oder X und $X_1$ gemeinsam eine Bindung darstellen, und worin $X_2$ Thio ist, oder ein pharmazeutisch verwendbares Salz derselben mit einer Base und/oder als Wirkstoffkomponente (B) das 2,6-Bis(diäthanolamino)-4,8-dipiperidino-pyrimido-[5,4]pyrimidin oder ein pharmazeutisch

verwendbares Säureadditionssalz davon.

4. Pharmazeutische Präparate gemäss Anspruch 1, die als Komponente A 1,2-Diphenyl-4-(2-phenylthioäthyl)-pyrazolidin-3,5-dion, 2-(2-Phenylthioäthyl)-malonsäure-N,N'-diphenylhydrazid oder 2-(2-Phenylsulfinyläthyl)-malonsäure-N,N'-diphenylhydrazid oder einen $C_1$-$C_4$-Alkylester bzw. ein Alkalimetallsalz davon und als Komponente B 2,6-Bis(diäthanolamino)-4,8-dipiperidino-pyrimdio-[5,4-d]pyrimidin oder das 2,6-Bis(diäthanolamino)-4-piperidino-pyrimido-[5,4-d]pyrimidin oder ein Säureadditionssalz davon enthalten.

5. Pharmazeutische Präparate gemäss Anspruch 1, die als Komponente A 1,2-Diphenyl-4-(2-phenylthioäthyl)-pyrazolidin-3,5-dion oder 2-(2-Phenylthioäthyl)-malonsäure-N,N'-diphenylhydrazid oder einen $C_1$-$C_4$-Alkylester bzw. ein Alkalimetallsalz davon und als Komponente B 2,6-Bis(diäthanolamino)-4,8-dipiperidino-pyrimido-[5,4-d]pyrimidin oder ein Säureadditionssalz davon enthalten.

6. Pharamzeutische Präparate gemäss Anspruch 1, die als Komponente A 2-(2-Phenylthioäthyl)-malonsäure-N,N'-diphenylhydrazid oder dessen Natriumsalz und als Komponente B 2,6-Bis(diäthanolamino)-4,8-dipiperidino-pyrimido-[5,4-d]pyrimidin enthalten.

7. Pharmazeutische Präparate gemäss Anspruch 1, die als Komponente A 2-(2-Phenylsulfinyläthyl)-malonsäure-N,N'-diphenylhydrazid oder dessen Natriumsalz und als Komponente B 2,6-Bis(diäthanolamino)-4,8-dipiperidino-pyrimido-[5,4-d]pyrimidin enthalten.

8. Pharmazeutische Präparate gemäss einem der Ansprüche 2, 4 und 7, dadurch gekennzeichnet, dass das Gewichtsverhältnis A:B von etwa 5:1 bis 1:5, vor allem etwa 2:1 bis 1:2, beträgt.

9. Pharamzeutische Präparate gemäss einem der Ansprüche 1, 3, 5 und 6, dadurch gekennzeichnet, dass das Gewichtsverhältnis A:B von etwa 5:1 bis 1:5, vor allem etwa 2:1 bis 1:2, beträgt.

10. Pharmazeutische Präparate gemäss einem der Ansprüche 2, 4 und 7, dadurch gekennzeichnet, dass das Gewichtsverhältnis A:B von etwa 1,5:1 bis 1:5, vor allem etwa 1:1 bis 1:2, beträgt.

11. Pharmazeutische Präparate gemäss einem der Ansprüche 1, 3, 5 und 6, dadurch gekennzeichnet, dass das Gewichtsverhältnis A:B von etwa 1,5:1 bis 1:5, vor allem etwa 1:1 bis 1:2, beträgt.

12. Pharmazeutische Präparate gemäss einem der Ansprüche 2, 4 und 7, dadurch gekennzeichnet, dass diese je etwa 10 bis 100 mg der Komponente A und etwa 25 bis 150 mg der Komponente B enthalten.

13. Pharmazeutische Präparate gemäss einem der Ansprüche 1, 3, 5 und 6, dadurch gekennzeichnet, dass diese je etwa 10 bis 100 mg der Komponente A und etwa 25 bis 150 mg der Komponente B, enthalten.

14. Pharmazeutische Präparate gemäss einem der Ansprüche 2, 4 und 7, dadurch gekennzeichnet, dass diese etwa 10 bis 75 mg, vor allem etwa 20 bis 60 mg, der Komponente A und etwa 25 bis 100 mg, vor allem etwa 25 bis 75 mg der Komponente B, enthalten.

15. Pharmazeutische Präparate gemäss einem der Ansprüche 1, 3, 5 und 6, dadurch gekennzeichnet, dass diese etwa 10 bis 75 mg, vor allem etwa 20 bis 60 mg, der Komponente A und etwa 25 bis 100 mg, vor allem etwa 25 bis 75 mg der Komponente B, enthalten.

16. Die in den Beispielen 1 bis 7 beschriebenen pharmazeutischen Präparate.

17. Die in den Beispielen 8 und 9 beschriebenen pharmazeutischen Präparate.

18. Verwendung von Präparaten gemäss einem der Ansprüche 2, 4, 7, 8, 10, 12 und 14 zur Behandlung thrombotischer Erkrankungen, insbesondere der Coronargefässe.

19. Verwendung von Präparaten gemäss einem der Ansprüche 3, 5, 6, 9, 11, 13 und 15 zur Behandlung thrombotischer Erkrankungen, insbesondere der Coronargefässe.

0019586

Europäisches Patentamt

## EUROPÄISCHER RECHERCHENBERICHT

Nummer der Anmeldung
EP 80 81 0151

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | EP - A - 0 000 774 (DR. KARL THOMAE GmbH)<br>* Ansprüche 1-4 *<br>-- | 1,9,11 13,15 |
| X | UNLISTED DRUGS, Band 29, November 1977, Chatham, N.J., US, "Asasantin"<br>* Seite 177q *<br>-- | 1 |
| X | US - A - 3 966 923 (HUBERT SERRE)<br>* Spalte 1, Zeilen 32-50; Spalte 3, Zeile 50 bis Spalte 6, Zeile 10; Ansprüche 1-18 *<br>-- | 1,9, 11,13, 15 |
| A | MARTIN NEGWER: "Organisch-Chemische Arzneimittel und ihre Synonyma", 5. Auflage, Band II, 1978, Akademie-Verlag Berlin, DE, Seiten 940-941<br>* Seite 940, Nr. 5061,5062; Seite 941, Nr. 5066 *<br>-- | 2-5,7 |
| A | DE - A - 2 023 415 (BYK-GULDEN LOMBERG CHEMISCHE FABRIK GmbH)<br>* Seiten 19-21; Ansprüche 1-3 *<br>----- | 2-4,7 |

KLASSIFIKATION DER ANMELDUNG (Int.Cl. 3)

A 61 K 45/06
31/505 //
(A 61 K 31/505
31/415) //
(A 61 K 31/505 //
31/19) //
(A 61 K 31/505
31/22)

RECHERCHIERTE SACHGEBIETE (Int. Cl. 3)

A 61 K 31/505

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 28-07-1980 | BRINKMANN |